# EUROPÄISCHE PATENTSCHRIFT

(11) **EP 0 299 277 B1**
(45) Veröffentlichungstag und Bekanntmachung des Hinweises auf die Patenterteilung: **18.10.1995**
(21) Anmeldenummer: 88110324.6
(22) Anmeldetag: 29.06.1988
(51) Int. Cl.: C07D 213/73, C07D 213/61, C07D 213/64, C07D 213/78, C07D 213/80

(54) **Verfahren zur Herstellung von substituierten Pyridylalkylketonen**
Process for the preparation of substituted pyridyl-alkyl ketones
Procédé pour la préparation de pyridylalkylcétones substituées

(30) Priorität: 11.07.1987 DE 3723070
(43) Veröffentlichungstag der Anmeldung: 18.01.1989
(73) Patentinhaber: BAYER AG, 51368 Leverkusen (DE)
(72) Erfinder: Lindel, Hans Dr., D-5090 Leverkusen 1 (DE); Hallenbach, Werner Dr., D-4018 Langenfeld (DE)

(56) Entgegenhaltungen:
- US-A- 4 358 455
- CHEMICAL ABSTRACTS, Band 80, Nr. 3, 21. Januar 1974, Columbus, Ohio, USA W. KORYTNYK et al. "Guanylhydrazones with potential antileukemic activity" Seite 11, Spalte 2, Zusammenfassung-Nr. 10 260a & J. Med. Chem., 16(8), 959-61 (1973)
- CHEMICAL ABSTRACTS, Band 95, Nr. 11, 14. September 1981, Columbus, Ohio, USA F. L. SETLIFF et al. "Some methyl 2,5- and 5,6 -dihalonicotinates" Seite 628, Spalte 1, Zusammenfassung-Nr. 97 527z & J. Chem. Eng. Data, 26(3), 332-33 (1981)

## Beschreibung

Die vorliegende Erfindung betrifft ein neues Verfahren zur Herstellung von substituierten Pyridylalkylketonen, Zwischenprodukte zur Durchführung dieses Verfahrens und deren Herstellung.

Pyridylalkylketone und ihre Verwendung als Zwischenprodukte zur Herstellung von Pyridylethanolaminen sind in einer nicht vorveröffentlichten Anmeldung der Anmelderin (Deutsche Patentanmeldung P 3 615 293.5) beschrieben.

Nach dem dort beschriebenen Verfahren werden sie erhalten, indem man Nikotinsäurealkylester mit Essigsäurealkylester in einer Claisen'schen Esterkondensation umsetzt und den so erhaltenen Pyridoylessigester verseift und decarboxyliert.

Die Pyridylethanolamine sind Wirkstoffe zur Förderung der Leistung von Tieren.

Es wurde nun gefunden
1. Verfahren zur Herstellung von Pyridylalkylketonen der Formel (I) 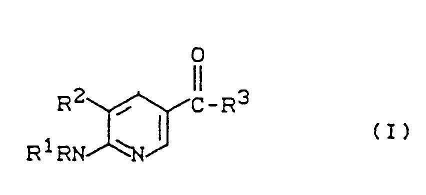 in welcher
   - R und R¹: unabhängig voneinander für Wasserstoff, C₁₋₃-Alkanoyl oder C₁₋₃-Alkyl stehen oder gemeinsam mit dem angrenzenden Stickstoffatom einen gegebenenfalls substituierten gesättigten oder ungesättigten heterocyclischen Rest aus der Reihe Pyrrol, Pyrrolidin, Piperidin, Morpholin.
   - R²: für Halogen steht,
   - R³: für Methyl oder Ethyl steht,
   das dadurch gekennzeichnet ist, daß man Verbindungen der Formel (II) 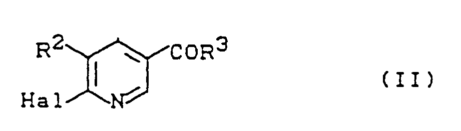 in welcher
   - R² und R³: die oben angegebene Bedeutung besitzen,
   - Hal: für Halogen steht,
   mit Aminen der Formel (III)

   H-NR R¹ (III)

   in welcher
   R und R¹ die oben angegebene Bedeutung haben in Gegenwart inerter organischer Verdünnungsmittel bei Temperaturen von 20 - 250 °C umsetzt.
2. Die Verbindungen der Formel (II) 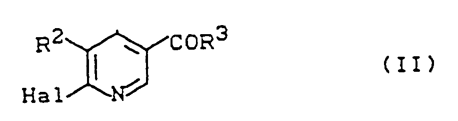 in welcher
   - Hal und R²: für Halogen stehen,
   - R³: für Methyl oder Ethyl steht.
3. Verfahren zur Herstellung der Verbindungen der Formel (II) 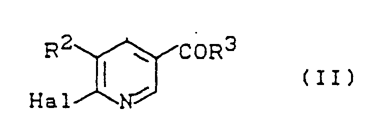 in welcher
   - Hal, R², R³: die oben angegebene Bedeutung haben,
   das dadurch gekennzeichnet ist, daß man Verbindungen der Formel (IV) 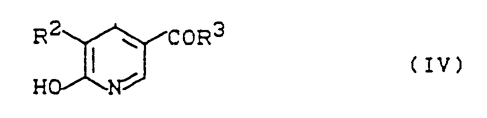 in welcher
   - R² und R³: die oben angegebene Bedeutung haben
   mit Phosphoroxyhalogeniden oder Phosgen in Gegenwart einer Hilfsbase in einem inerten organischen Verdünnungsmittel bei Temperaturen von 20 - 280 °C halogeniert.
4. Die Verbindungen der Formel (IV) 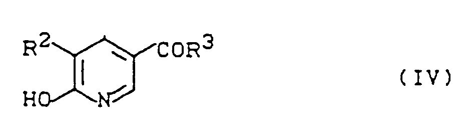 in welcher
   - R² und R³: die oben bei den Verbindungen der Formel (I) angegebene Bedeutung haben.
5. Verfahren zur Herstellung der Verbindungen der Formel (IV) 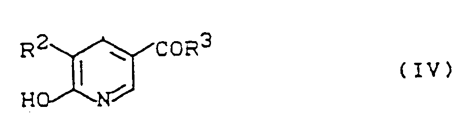 in welcher
   - R² und R³: die oben angegebene Bedeutung haben,
   dadurch gekennzeichnet, daß man Verbindungen der Formel (V) 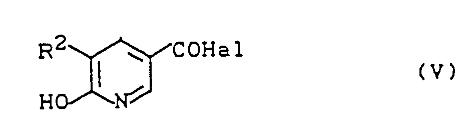 in welcher
   - R²: die oben angegebene Bedeutung hat und
   - Hal: für Halogen steht,
   mit Verbindungen der Formel (VI) 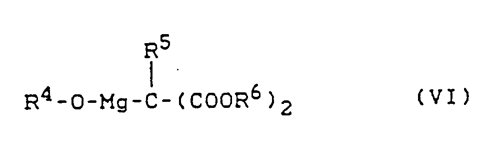 in welcher
   - R⁴: für C₁₋₃-Alkyl,
   - R⁵: für Wasserstoff oder C₁₋₂-Alkyl,
   - R⁶: für C₁₋₃-Alkyl steht
   in einem inerten organischen Verdünnungsmittel bei Temperaturen von 20 - 150°C umsetzt, anschließend in Gegenwart von Säuren oder Basen verseift und decarboxyliert.
6. Die Verbindungen der Formel (V) 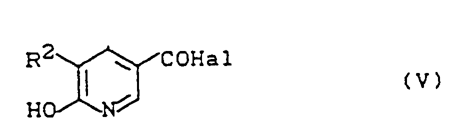 in welcher
   - Hal und R²: für Halogen stehen.
7. Verfahren zur Herstellung der Verbindungen der Formel (V) 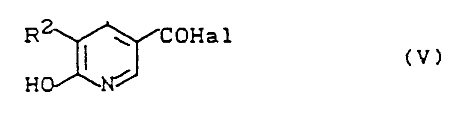 in welcher
   - Hal und R²: für Halogen stehen,
   dadurch gekennzeichnet, daß man Verbindungen der Formel (VII) 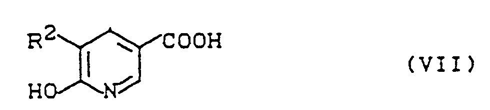 in welcher
   - R²: für Halogen steht
   mit 0,1 - 1,5 Äquivalenten eines anorganischen Säurehalogenids als Halogenierungsmittel bei Temperaturen von 20 - 100 °C umsetzt.

Bevorzugt werden nach den neuen Verfahren Verbindungen der Formel (I) hergestellt, in welcher
- R: für Wasserstoff oder C₁-C₃-Alkyl steht,
- R¹: für Wasserstoff, C₁-C₃-Alkyl oder C₁-C₃-Alkanoyl steht,
oder R und R¹ gemeinsam mit dem Stickstoffatom, an das sie gebunden sind, für einen heterocyclischen Rest stehen,
- R²: für Chlor oder Brom steht und
- R³: für Methyl oder Ethyl steht.

Besonders bevorzugt werden Verbindungen der Formel (I) hergestellt, in der
- R: für Wasserstoff oder Methyl steht,
- R¹: für Wasserstoff, Methyl oder Acetyl steht
oder R und R¹ gemeinsam mit dem Stickstoffatom für einen 5- oder 6-gliedrigen, gegebenenfalls weitere Heteroatome enthaltenden Ring wie Pyrrolidonyl, Pyrryl, Piperidinyl oder Morpholinyl stehen,
- R²: für Chlor steht,
- R³: für Methyl oder Ethyl steht.

Die Verbindungen der Formel (I), in denen mindestens einer der Reste R und R¹ für Wasserstoff steht, können auch in den folgenden tautomeren Formen vorliegen. 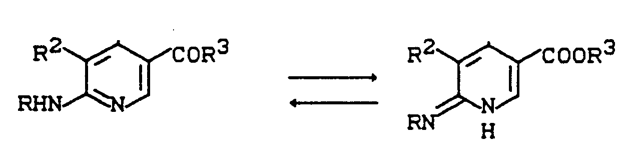
Im einzelnen seien folgende Verbindungen der Formel (I) genannt:
2-Amino-3-chlor-5-acetylpyridin
2-Amino-3-chlor-5-propionylpyridin
2-Amino-3-brom-5-acetylpyridin
2-Amino-3-brom-5-propionylpyridin
2-Methylamino-3-chlor-5-acetylpyridin
2-Acetylamino-3-chlor-5-acetylpyridin
2-Acetylamino-3-brom-5-acetylpyridin
2-(N-Pyrrolo)-3-chlor-5-acetylpyridin
2-(N-Morpholino)-3-chlor-5-acetylpyridin
Setzt man bei Verfahren 1) als Verbindung der Formel (II) 2,3-Dibrom-5-propionylpyridin und als Amin der Formel (III) Methylamin ein, laßt sich das Verfahren durch folgendes Schema darstellen: 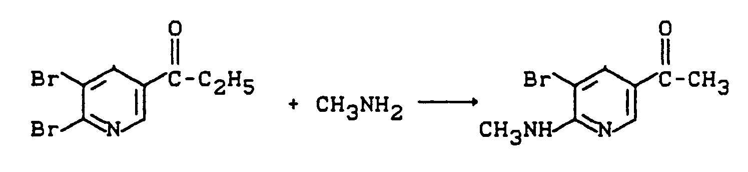
Amine der Formel (III) sind bekannt, Bevorzugt sind Amine der Formel (III), in welcher R und R¹ die bei den Verbindungen der Formel (I) angegebenen bevorzugten Bedeutungen haben, Im einzelnen seien folgende Amine der Formel (III) genannt:
Ammoniak, Methylamin, Ethylamin, n- und iso-Propylamin, Dimethylamin, Diethylamin, Methylethylamin, Pyrrol, Pyrrolidin, Piperidin, Morpholin, Methylanilin, Ethylanilin,

Verbindungen der Formel (II) sind neu, Ihre Herstellung erfolgt nach dem weiter unten beschriebenen Verfahren, Bevorzugt sind Verbindungen der Formel (II), in welcher R² und R³ die bei den Verbindungen der Formel (I) angegebenen bevorzugten Bedeutungen haben und Hal für Chlor oder Brom steht.

Im einzelnen seien genannt:
2,3-Dichlor-5-acetylpyridin,
2,3-Dichlor-5-propionylpyridin,
2-Chlor-3-brom-5-acetylpyridin,
2-Chlor-3-brom-5-propionylpyridin,
2,3-Dibrom-5-propionylpyridin,
2,3-Dibrom-5-acetylpyridin.

Verfahren 1) wird durchgeführt, indem man die Verbindung der Formel (II) mit der 2- bis 5-fachen, bevorzugt 2- bis 3-fachen molaren Menge Amin der Formel (III) gegebenenfalls in einem Verdünnungsmittel umsetzt.

Als Verdünnungsmittel können alle inerten organischen Lösungsmittel verwendet werden. Hierzu gehören insbesondere gegebenenfalls halogenierte aliphatische oder aromatische Kohlenwasserstoffe wie Pentan, Hexan, Heptan, Cyclohexan, Benzol, Toluol, Dichlormethan, Chloroform, Dichlorethan, Chlorbenzol, weiterhin Ether wie Diethylether, Tetrahydrofuran oder Dioxan, ebenso Amide wie Dimethylformamid, ebenso Wasser.

Die Reaktion wird bei Temperaturen von 20°C bis 250°C durchgeführt.

Es wird bei Normaldruck oder unter erhöhtem Druck gearbeitet,

Setzt man bei Verfahren 3) als Verbindung der Formel (IV) 2-Hydroxy-3-brom-5-propionylpyridin ein, läßt sich das Verfahren durch folgendes Schema darstellen: 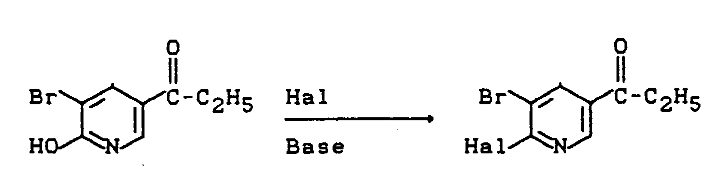
Die Verbindungen der Formel (IV) sind neu. Sie werden nach dem weiter unten beschriebenen Verfahren hergestellt. Die Substituenten R² und R³ besitzen bevorzugt die bei Verbindungen der Formel (I) angegebenen bevorzugten Bedeutungen. Im einzelnen seien folgende Verbindungen der Formel (IV) genannt:
2-Hydroxy-3-chlor-5-acetylpyridin
2-Hydroxy-3-chlor-5-propionylpyridin
2-Hydroxy-3-brom-5-acetylpyridin
2-Hydroxy-3-brom-5-propionylpyridin.

Verfahren 3) wird durchgeführt, indem man eine Verbindung der Formel (IV) in Gegenwart der äquimolaren Menge einer Hilfsbase mit etwa der äquimolaren Menge eines Halogenierungsmittels in einem Verdünnungsmittel umsetzt, Als Verdünnungsmittel können alle inerten organischen Lösungsmittel verwendet werden. Hierzu zählen gegebenenfalls halogenierte aliphatische oder aromatische Kohlenwasserstoffe wie Pentan, Hexan, Heptan, Cyclohexan, Benzol, Toluol, Dichlormethan, Chloroform, Dichlorethan, Chlorbenzol, Dichlorbenzol; weiterhin Ether wie Diethylether und Tetrahydrofuran.

Als Halogenierungsmittel dienen vorzugsweise Phosphoroxyhalogenide wie Phosphoroxychlorid und Phosphoroxybromid sowie Phosgen.

Als Hilfsbasen bei der Reaktion werden tertiäre Amine, wie Trialkylamine, Dialkylarylamine, Alkyldiarylamine sowie Pyridin verwendet.

Die Umsetzung wird bei Temperaturen von 20°C bis 200°C, bevorzugt bei 50°C bis 150°C durchgeführt.

Es wird unter Normaldruck oder erhöhtem Druck gearbeitet.

Setzt man bei Verfahren 5) als Verbindung der Formel (V) 5-Brom-6-hydroxynikotinsäurechlorid und als Verbindung der Formel (VI) Methoxymagnesiummalonsäuredimethylester ein, läßt sich Verfahren 5) durch das folgende Reaktionsschema darstellen: 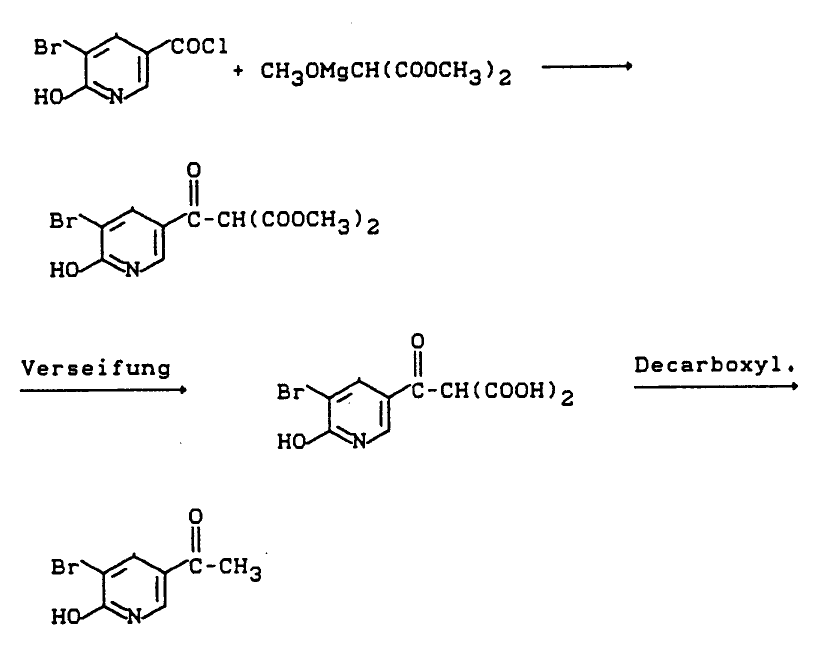
Verbindungen der Formel (V) sind neu. Ihre Herstellung wird weiter unter beschrieben. Die Substituenten R² und Hal besitzen bevorzugt die weiter oben angegebene Bedeutungen. Im einzelnen seien folgende Verbindungen der Formel (V) genannt:
5-Brom-6-hydroxynikotinsäurechlorid,
5-Chlor-6-hydroxynikotinsäurechlorid.

Verbindungen der Formel (VI) sind bekannt (Org. Synth. Coll. Vol. IV (1963), 285; Ber. Dt. Chem. Ges. 67 (1934), 935).

Im einzelnen seien folgende Verbindungen der Formel (VI) genannt:
Methoxymagnesiummalonsäuredimethylester,
Ethoxymagnesiummalonsäurediethylester,
Ethoxymagnesiummethylmalonsäurediethylester.

Das Verfahren wird durchgeführt, indem man äquimolare Mengen der Verbindungen der Formeln (V) und (VI) in einem Verdünnungsmittel zur Reaktion bringt und anschließend verseift, wobei die β-Ketodicarbonsäure decarboxyliert. Als Verdünnungsmittel dienen alle inerten organischen Lösungsmittel. Hierzu zählen gegebenenfalls halogenierte aliphatische und aromatische Kohlenwasserstoffe wie Pentan, Hexan, Benzol, Toluol, Dichlormethan, Chloroform, Chlorbenzol; Ether wie Diethylether, Tetrahydrofuran; Alkohole wie Methanol, Ethanol. Die Reaktion wird bei Temperaturen von 20°C bis 150°C, bevorzugt bei der Siedetemperatur des verwendeten Lösungsmittels durchgeführt.

Die Verseifung wird mit anorganischen Säuren wie Salzsäure oder Schwefelsäure, organischen Carbonsäuren wie Essigsäure oder Propionsäure durchgeführt. Es können auch Mischungen von anorganischen und organischen Säuren verwendet werden. Eine Verseifung mit alkoholischen und wäßrigen Lösungen von Basen wie Alkalihydroxiden und Erdalkalihydroxiden, -carbonaten und -hydrogencarbonaten ist auch möglich. Im einzelnen seien als Basen genannt: Natrium-, Kalium-, Bariumhydroxid, Natrium- und Kaliumcarbonat.

Setzt man bei Verfahren 7) als Carbonsäure der Formel (VII) 5-Brom-6-hydroxy-nikotinsäure und als Halogenierungsmittel Thionylchlorid ein, laßt sich die Reaktion durch das folgende Schema darstellen: 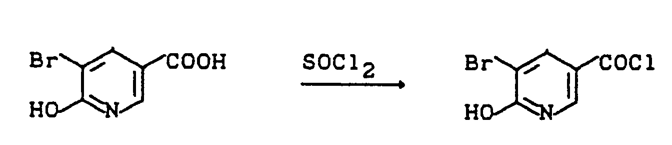
Die Verbindungen der Formel (VII) sind bekannt (EP-OS 136 593). Bevorzugt sind Verbindungen der Formel (VII), in denen R² für Chlor oder Brom steht.

Als Halogenierungsmittel werden anorganische Säurechloride verwendet, Beispielhaft seien genannt: Phosphoroxychlorid, Phosphorpentachlorid, Thionylchlorid.

Die Reaktion wird durchgeführt, indem man eine substituierte Nikotinsaure der Formel (VII) mit 0,1 bis 1,5 Äquivalenten des anorganischen Säurechlorids, gegebenenfalls in einem Verdünnungsmittel, behandelt,

Die Umsetzung wird bei Temperaturen von 20°C bis 100°C durchgeführt, es wird bevorzugt bei Normaldruck gearbeitet,

Als Verdünnungsmittel können alle inerten organischen Lösungsmittel verwendet werden, Hierzu zählen insbesondere aliphatische und aromatische gegebenenfalls halogenierte Kohlenwasserstoffe wie Pentan, Hexan, Cyclohexan, Benzol, Toluol, Methylenchlorid, Chloroform, Chlorbenzol; Ether wie Diethylether, Tetrahydrofuran, Dioxan sowie Phosphoroxychlorid.

Bevorzugt wird ohne Verdünnungsmittel gearbeitet.

Die nach dem erfindungsgemäßen Verfahren 1) erhältlichen substituierten Pyridylalkylketone der Formel (I) werden zur Herstellung von Pyridylethanolaminderivaten verwendet. Dazu werden die Acetylpyridine mit elementarem Halogen oder mit Kupferhalogeniden umgesetzt. Die dabei erhältlichen Halogenmethylpyridylketone werden anschließend reduziert und die dabei erhaltenen Pyridylhalogenethanole mit Aminen umgesetzt. Diese Reaktion laßt sich durch folgendes Formelschema illustrieren: 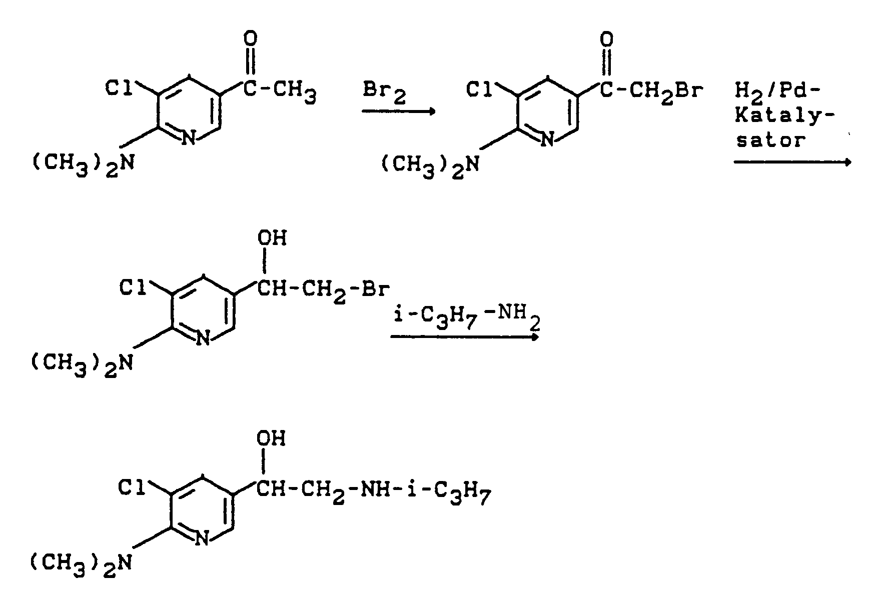
Die Halogenmethylketone können auch zunächst mit Aminen umgesetzt und anschließend reduziert werden.

Die Durchführung dieser Reaktionen ist in der oben erwähnten nicht vorveröffentlichten Anmeldung der Anmelderin (Deutsche Patentanmeldung P 3 615 293.5) beschrieben.

### Herstellungsbeispiele

### Beispiel für Verfahren 1

### Beispiel 1

### 2-Amino-3-chlor-5-acetylpyridin

1,9 g (10 mmol) 2,3-Dichlor-5-acetylpyridin werden in einer Mischung von 80 ml Tetrahydrofuran und 20 ml konzentriertem wäßrigem Ammoniak im Autoklaven 8 Stunden bei 170°C erhitzt. Nach Abdampfen des Tetrahydrofurans wird mit Wasser verdünnt, auf pH 5 gestellt und mit Essigester extrahiert.
Ausbeute: 1,65 g (97 %), Fp.: 188°C.

### Beispiel für Verfahren 3

### Beispiel 2

### 2,3-Dichlor-5-acetylpyridin

3.4 g (20 mmol) 2-Hydroxy-3-chlor-5-acetylpyridin werden mit 3,7 g (24 mmol) Phosphoroxychlorid und 2,4 g (20 mmol) N,N-Dimethylanilin in 75 ml Chlorbenzol 1,5 Stunden bei 100°C gerührt. Die Mischung wird in 200 ml Eiswasser eingerührt und mit Essigester extrahiert. Nach Trocknen und Eindampfen erhält man 3,6 g (95 %) 2,3-Dichlor-5-acetylpyridin, Fp.: 80°C.

### Beispiel für Verfahren 5

### Beispiel 3

### 2-Hydroxy-3-chlor-5-acetylpyridin

Zur siedenden Lösung von 8,55 g (37,5 mmol) Ethoxymagnesium-malonsäurediethylester (hergestellt nach Org. Synth. Coll. Vol. IV (1963), 285) in 120 ml abs, Tetrahydrofuran werden 7,2 g (37,5 mmol) 5-Chlor-6-hydroxynikotinsäurechlorid in 10 ml abs. Tetrahydrofuran gegeben, die Mischung wird 2 Stunden unter Rückfluß erhitzt. Nach Neutralisation mit 2N Schwefelsäure wird die organische Phase abgetrennt und eingedampft. Der Rückstand wird in einer Mischung aus 30 ml Eisessig, 20 ml Wasser und 5 ml konz. Schwefelsäure 4 Stunden unter Rückfluß erhitzt. Der Ansatz wird in Eiswasser eingerührt, auf pH 4 gestellt und mit Essigester extrahiert. Nach Trocknen und Eindampfen erhalt man 6 g (93 %) der Titelverbindung, Fp.; 188 bis 189°C.

### Beispiel für Verfahren 7

### Beispiel 4

### 5-Chlor-6-hydroxynikotinsäurechlorid

6,3 g (36 mmol) 5-Chlor-6-hydroxynikotinsäure werden in 60 ml Thionylchlorid 4 Stunden unter Rückfluß erhitzt. Nach Abziehen der flüchtigen Bestandteile wird mit 50 ml Toluol versetzt und wieder eingedampft. Farblose Kristalle, Ausbeute 6,9 g (quant.), Fp.: >250°C
Beispiele für die weitere Umsetzung der substituierten Pyridylalkylketone zu substituierten Pyridylethanolaminen

### Beispiel a (Halogenierung der Pyridylalkylketone)

### 2-Amino-3-chlor-5-pyridyl-(brommethyl)keton

Zu einer Lösung von 17,05 g (0,1 mol) 2-Amino-3-chlor-5-acetylpyridin in einer Mischung von 19,3 g Bromwasserstoff (47 %ige wäßrige Lösung; 0,11 mol) und 500 ml Eisessig werden in 16 g (0,1 mol) Brom getropft. Man rührt zwei Stunden nach, stellt die Mischung auf pH 8 und extrahiert mit Essigester. Nach Trocknen und Eindampfen erhält man 18,5 g (74 %) der Titelverbindung, Fp. 134°C.

### Beispiel b (Umsetzung der Pyridylhalogenalkylketone mit Aminen)

### 2-Amino-3-chlor-5-pyridyl-(isopropylaminomethyl)keton

In eine Lösung von 11,8 g (0,2 mol) Isopropylamin in 150 ml Methanol werden bei 0°C portionsweise 9,98 g (0,04 mol der unter Beispiel a hergestellten Verbindung eingetragen. Man läßt auf Raumtemperatur kommen, rührt 2 Stunden nach und dampft ein. Der Rückstand wird in Puffer pH 5 aufgenommen und mit Ether gewaschen. Die wäßrige Phase wird auf pH 9 gestellt und mit Essigester extrahiert. Nach Trocknen und Eindampfen werden 6,8 g (75 %) der Titelverbindung als amorphes Pulver erhalten.

### Beispiel c (Reduktion der Pyridylaminoalkylketone)

### 1-(2-Amino-3-chlor-5-pyridyl)-2-isopropylaminoethanol

Zur Lösung von 2,28 g (10 mmol) der nach Beispiel b hergestellten Verbindung in 50 ml Methanol werden bei 0°C portionsweise 0,38 g (10 mmol) Natriumborhydrid gegeben. Man läßt auf Raumtemperatur kommen, stellt mit verdünnter Salzsäure auf pH 1 und dampft ein. Der Rückstand wird in Wasser aufgenommen und mit Ether gewaschen. Dann wird auf pH 10 gestellt und mit Essigester extrahiert. Nach Trocknen und Eindampfen erhält man 2,1 g (92 %) dar Titelverbindung, Fp. 146°C.

## Patentansprüche

1. Verfahren zur Herstellung von Pyridylalkylketonen der Formel (I) 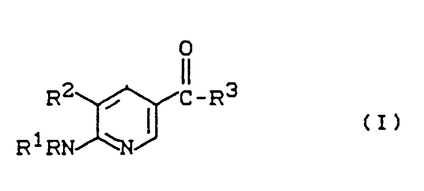 in welcher
R und R¹ unabhängig voneinander für Wasserstoff, C₁₋₃-Alkanoyl oder C₁₋₃-Alkyl stehen oder gemeinsam mit dem angrenzenden Stickstoffatom einen gegebenenfalls substituierten gesättigten oder ungesättigten heterocyclischen Rest aus der Reihe Pyrrol, Pyrrolidin, Piperidin, Morpholin.
R² für Halogen steht,
R³ für Methyl oder Ethyl steht,
das dadurch gekennzeichnet ist, daß man Verbindungen der Formel (II) 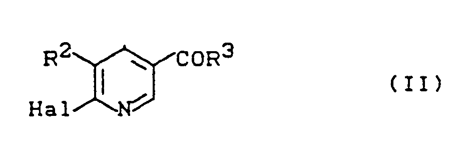 in welcher
R² und R³ die oben angegebene Bedeutung besitzen,
Hal für Halogen steht,
mit Aminen der Formel (III)
H-NR R¹ (III)
in welcher
R und R¹ die oben angegebene Bedeutung haben in Gegenwart inerter organischer Verdünnungsmittel bei Temperaturen von 20 - 250 °C umsetzt.

2. Die Verbindungen der Formel (II) 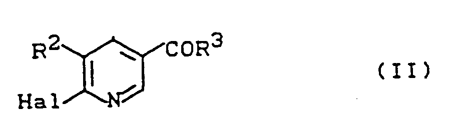 in welcher
Hal und R² für Halogen stehen,
R³ für Methyl oder Ethyl steht.

3. Verfahren zur Herstellung der Verbindungen der Formel (II) 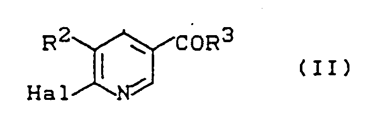 in welcher
Hal, R², R³ die in Anspruch 2 angegebene Bedeutung haben,
das dadurch gekennzeichnet ist, daß man Verbindungen der Formel (IV) 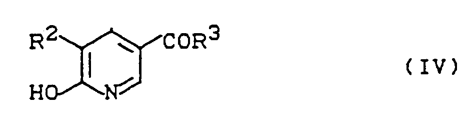 in welcher
R² und R³ die oben angegebene Bedeutung haben
mit Phosphoroxyhalogeniden oder Phosgen in Gegenwart einer Hilfsbase in einem inerten organischen Verdünnungsmittel bei Temperaturen von 20 - 280 °C halogeniert.

4. Die Verbindungen der Formel (IV) 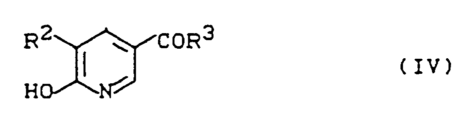 in welcher
R² und R³ die in Anspruch 1 bei den Verbindungen der Formel (I) angegebene Bedeutung haben.

5. Verfahren zur Herstellung der Verbindungen der Formel (IV) 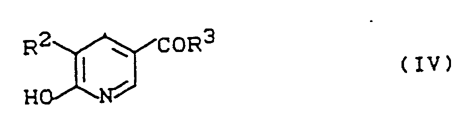 in welcher
R² und R³ die in Anspruch 1 angegebene Bedeutung haben,
dadurch gekennzeichnet, daß man Verbindungen der Formel (V) 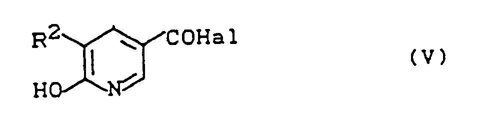 in welcher
R² die oben angegebene Bedeutung hat und
Hal für Halogen steht,
mit Verbindungen der Formel (VI) 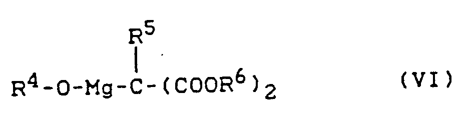 in welcher
R⁴ für C₁₋₃-Alkyl,
R⁵ für Wasserstoff oder C₁₋₂-Alkyl,
R⁶ für C₁₋₃-Alkyl steht
in einem inerten organischen Verdünnungsmittel bei Temperaturen von 20 - 150°C umsetzt, anschließend in Gegenwart von Säuren oder Basen verseift und decarboxyliert.

6. Die Verbindungen der Formel (V) 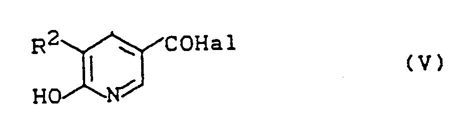 in welcher
Hal und R² für Halogen stehen.

7. Verfahren zur Herstellung der Verbindungen der Formel (V) 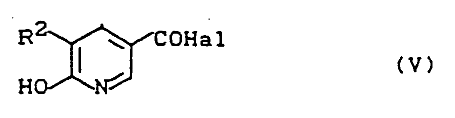 in welcher
Hal und R² für Halogen stehen,
dadurch gekennzeichnet, daß man Verbindungen der Formel (VII) 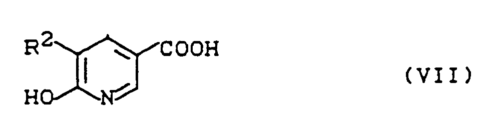 in welcher
R² für Halogen steht
mit 0,1 - 1,5 Äquivalenten eines anorganischen Säurehalogenids als Halogenierungsmittel bei Temperaturen von 20 - 100 °C umsetzt.

## Claims

1. Process for the preparation of pyridyl alkyl ketones of the formula (I) 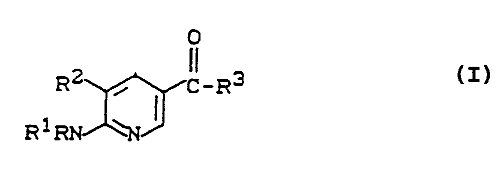 in which
R and R¹ independently of one another represent hydrogen, C₁₋₃-alkanoyl or C₁₋₃-alkyl or, together with the adjacent nitrogen atom, form an optionally substituted saturated or unsaturated heterocyclic radical from the series pyrrole, pyrrolidine, piperidine, morpholine,
R² represents halogen and
R³ represents methyl or ethyl,
which is characterized in that compounds of the formula (II) 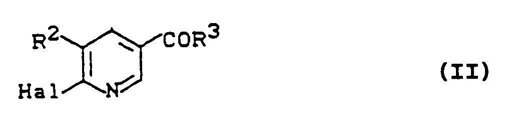 in which
R² and R³ have the abovementioned meaning and
Hal represents halogen,
are reacted with amines of the formula (III)
H-NR R¹ (III)
in which
R and R¹ have the abovementioned meaning in the presence of inert organic diluents at temperatures of 20-250°C.

2. Compounds of the formula (II) 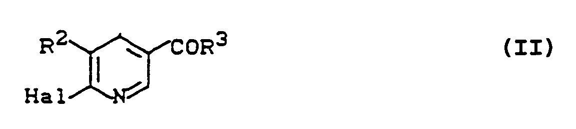 in which
Hal and R² represent halogen and
R³ represents methyl or ethyl.

3. Process for the preparation of the compounds of the formula (II) 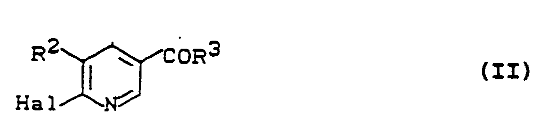 in which
Hal, R² and R³ have the meaning given in Claim 2,
which is characterized in that compounds of the formula (IV) 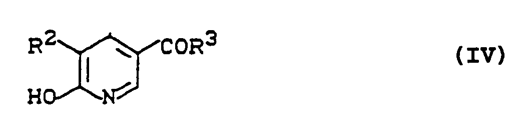 in which
R² and R³ have the abovementioned meaning
are halogenated with phosphorus oxyhalides or phosgene in the presence of an auxiliary base in an inert organic diluent at temperatures of 20 - 280°C.

4. Compounds of the formula (IV) 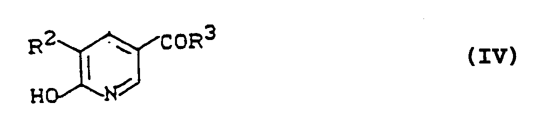 in which
R² and R³ have the meaning given in Claim 1 in the case of the compounds of the formula (I)

5. Process for the preparation of the compounds of the formula (IV) 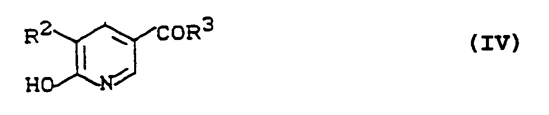 in which
R² and R³ have the meaning given in Claim 1,
characterized in that compounds of the formula (V) 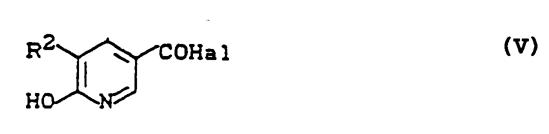 in which
R² has the abovementioned meaning and
Hal represents halogen,
are reacted with compounds of the formula (VI) 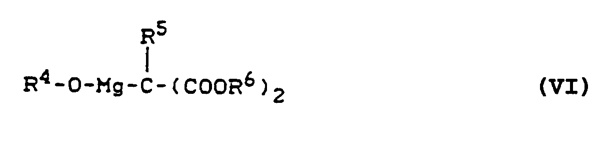 in which
R⁴ represents C₁₋₃-alkyl,
R⁵ represents hydrogen or C₁₋₂-alkyl and
R⁶ represents C₁₋₃-alkyl,
in an inert organic diluent at temperatures of 20 - 150°C, and the products are then hydrolysed and decarboxylated in the presence of acids or bases.

6. Compounds of the formula (V) 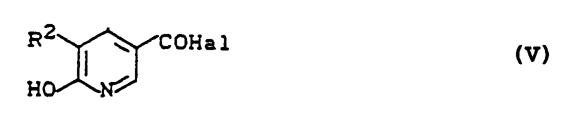 in which
Hal and R² represent halogen.

7. Process for the preparation of the compounds of the formula (V) 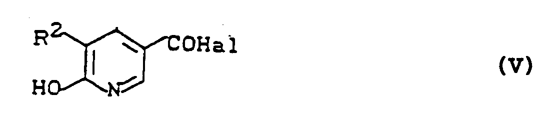 in which
Hal and R² represent halogen,
characterized in that compounds of the formula (VII) 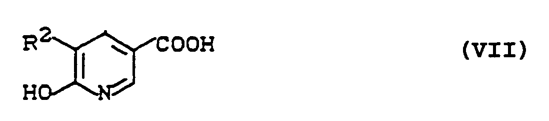 in which
R² represents halogen,
are reacted with 0.1 - 1.5 equivalents of an inorganic acid halide as halogenating agent at temperatures of 20 - 100°C.

## Revendications

1. Procédé de production de pyridylalkylcétones de formule (I) 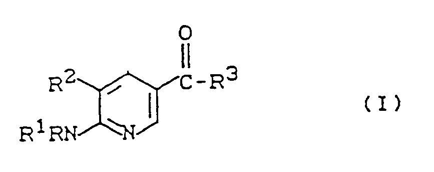 dans laquelle
R et R¹ représentent, indépendamment l'un de l'autre, de l'hydrogène, un groupe alcanoyle en C₁ à C₃ ou un groupe alkyle en C₁ à C₃, ou forment conjointement avec l'atome contigu d'azote un reste hétérocyclique saturé ou non saturé éventuellement substitué, de la série pyrrole, pyrrolidine, pipéridine, morpholine,
R² est un halogène,
R³ est un groupe méthyle ou éthyle,
caractérisé en ce qu'on fait réagir à des températures de 20-250°C des composés de formule (II) 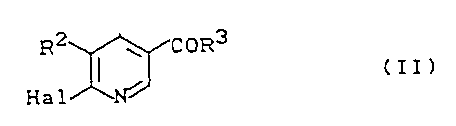 dans laquelle
R² et R³ ont la définition indiquée ci-dessus,
Hal représente un halogène,
avec des amines de formule (III)
H-NR R¹ (III)
dans laquelle
R et R¹ ont la définition indiquée ci-dessus,
en présence d'un diluant organique inerte.

2. Composés de formule (II) 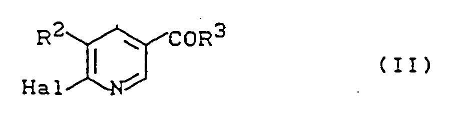 dans laquelle
Hal et R² représentent un halogène,
R³ est un groupe méthyle ou éthyle.

3. Procédé de production des composés de formule (II) 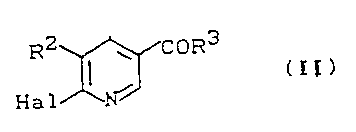 dans laquelle
Hal, R², R³ ont la définition indiquée dans la revendication 2,
caractérisé en ce qu'on halogène à des températures de 20 à 280°C des composés de formule (IV) 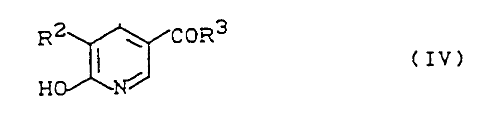 dans laquelle
R² et R³ ont la définition indiquée ci-dessus,
avec des oxyhalogénures de phosphore ou du phosgène en présence d'une base auxiliaire dans un diluant organique inerte.

4. Composés de formule (IV) 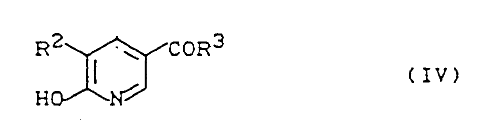 dans laquelle
R² et R³ ont la définition indiquée dans la revendication 1 pour les composés de formule (I).

5. Procédé de production des composés de formule (IV) 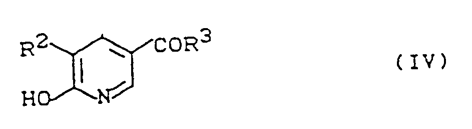 dans laquelle
R² et R³ ont la définition indiquée dans la revendication 1,
caractérisé en ce qu'on fait réagir à des températures de 20 à 150°C des composés de formule (V) 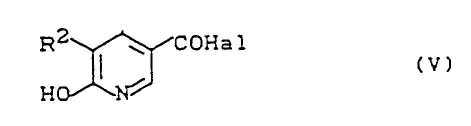 dans laquelle
R² a la définition indiquée ci-dessus et
Hal représente un halogène,
avec des composés de formule (VI) 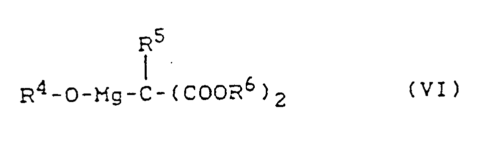 dans laquelle
R⁴ est un groupe alkyle en C₁ à C₃,
R⁵ est de l'hydrogène ou un groupe alkyle en C₁ ou C₂,
R⁶ est un groupe alkyle en C₁ à C₃,
dans un diluant organique inerte, puis on effectue une saponification et une décarboxylation en présence d'acides ou de bases.

6. Composés de formule (V) 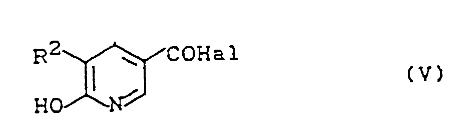 dans laquelle
Hal et R² représentent un halogène.

7. Procédé de production des composés de formule (V) 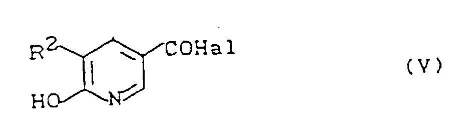 dans laquelle
Hal et R² représentent un halogène,
caractérisé en ce qu'on fait réagir à des températures de 20 à 100°C des composés de formule (VII) 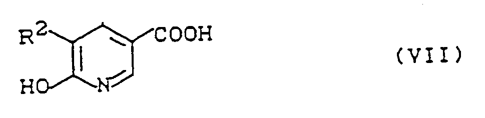 dans laquelle
R² représente un halogène,
avec, comme agent d'halogénation, de 0,1 à 1,5 équivalent d'un halogénure d'acide inorganique.
